# EUROPEAN PATENT APPLICATION

(11) **EP 1 958 936 A1**
(43) Date of publication of application: **20.08.2008**
(21) Application number: 07380038.5
(22) Date of filing: 14.02.2007
(51) Int. Cl.: C07D 215/18

(54) **Process for obtaining montelukast**

(71) Applicant: INKE, S.A., 08755 Castellbisbal (ES)
(72) Inventor: Pérez Andrés, Juan Antonio, 08980 Sant Feliu e Llobregat Barcelona (ES); Huguet Clotet, Juan, 08970 Sant Joan Despi Barcelona (ES); Dalmases Barjoan, Pere, 08980 Sant Feliu de Llobregat Barcelona (ES)
(74) Representative: ABG Patentes, S.L.

(57) **Abstract**

The present invention refers to a process for the synthesis of (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid or its salts, comprising the reaction between 7-Chloro-2-vinyl-quinoline and a compound of formula (I) 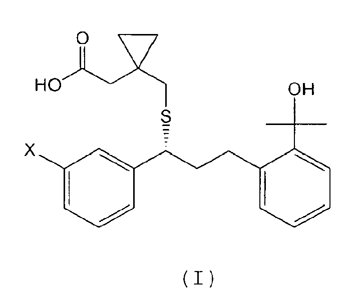 wherein X is a halogen atom or a group of formula - OSO₂R, wherein R is selected from the group consisting of CF₃, tolyl, methyl and F;
in the presence of a palladium based catalyst. The invention is also directed to intermediates compounds of the process.

## Description

### FIELD OF THE INVENTION

The present invention is directed to a process for the synthesis of Montelukast and salts thereof, and to intermediate compounds of said process.

### BACKGROUND

Montelukast or (1-{1-(R)-(E)-{3-[2-(7-Chloro-quinolin-2-yl)-vinyl]-phenyl}-3-[2-(1-hydroxy-1-methylethyl)-phenyl]-propylsulfanylmethyl}-cyclopropyl)-acetic acid has the following formula

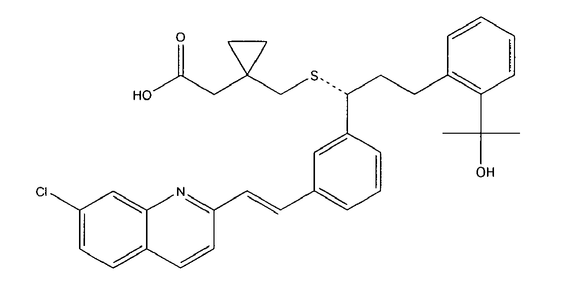

Sodium Montelukast is used in the treatment of asthma. It is comercialized under the name of SINGULAIR® (Merck) as oral tablets, chewable tablets and granules. Sodium Montelukast has the following structure:

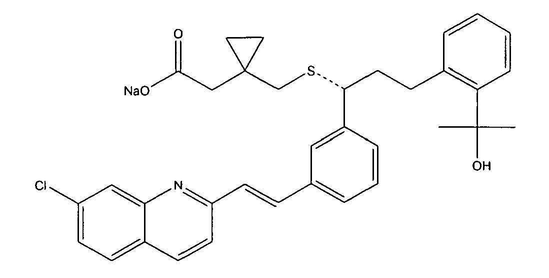

EP 480.717 B1 described for the first time a family of compounds wherein Sodium Montelukast was comprised. The synthesis described in said patent involved methyl esters such as methyl 2-[(3S)-[3-[(2E)-(7-chloroquinolyn-2-yl)ethenylphenyl]-3-hydroxipropyl]benzoate and comprised the coupling between methyl 1-(mercaptometyl)-cyclopropaneacetate and an appropriate mesilate produced in situ. The methyl ester of Montelukast was hydrolyzed into its acid and directly transformed into its corresponding sodium salt (Scheme 1). The tedious chromatographic purifications of the methyl esters and final products required makes the above process unsuitable for large scale production. Additionally, the yields obtained are poor.

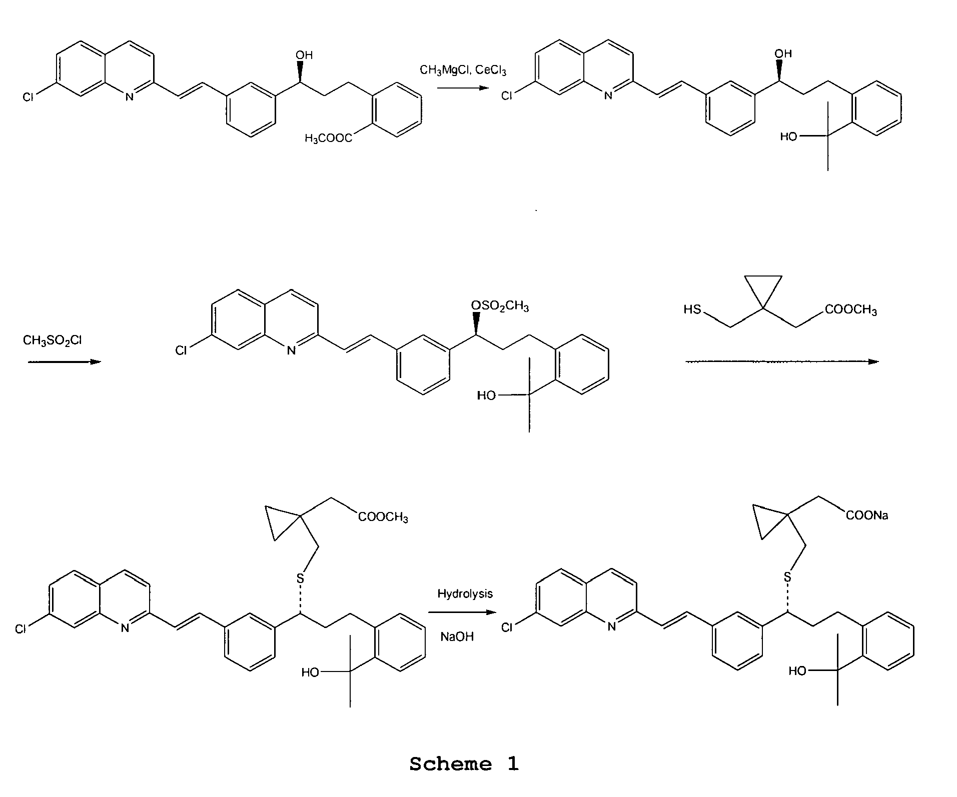

EP 737.186 B1 described an improved process for the synthesis of sodium Montelukast and dicyclohexylammonium Montelukast, which differed from the process described in EP 480.717 B1 in the use of the dilithium salt of 1-(mercaptomethyl)cyclopropaneacetic acid, instead of the methyl ester for the coupling reaction with the mesylate. Said mesylate had the same formula as in EP 480.717 B1 but was added in its crystalline form. The process directly yielded Montelukast in its acid form, which was further transformed into its dicyclohexylamine salt, which crystallizes in two different polymorphs (Form A and Form B). From said purified and crystalline dicyclohexylamine salt, Montelukast in its acid form was recovered by treatment with acid, and then the sodium salt was obtained by treatment of the free acid with a source sodium ions (Scheme 2).

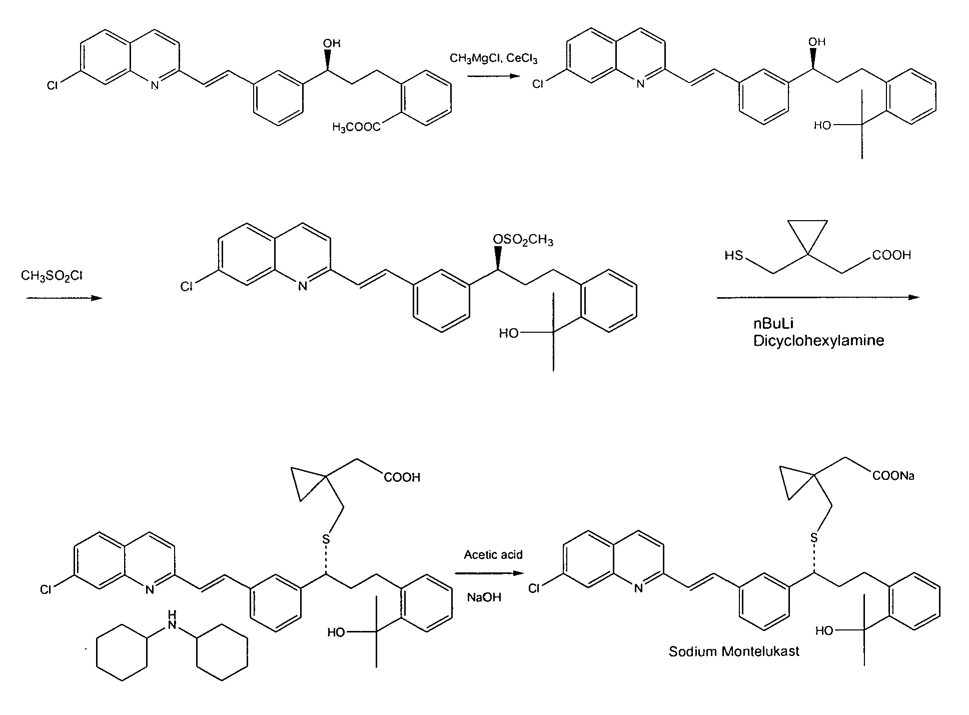

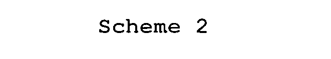

The procedure disclosed in EP 737.186 B1 requires some steps to be performed with temperatures under -25°C. Further, some of the intermediates used, such as the mesylate, are unstable. Additionally, highly flammable and dangerous chemicals, such as butyl lithium, are used. The above disadvantages make the process not convenient for the industrial scale.

The present invention provides a new process for the synthesis of Montelukast which overcomes the above mentioned problems.

### SUMMARY OF THE INVENTION

As mentioned above, a first aspect of the present invention is a process for the synthesis of Montelukast or (1-{1-(R)-(E)-{3-[2-(7-chloro-quinolin-2-yl)-vinyl]-phenyl} -3-[2-(1-hydroxy-1-methyl-ethyl)-phenyl]-propylsulfanyl methyl} - cyclopropyl)-acetic acid or its salts, comprising the reaction between 7-Chloro-2-vinyl-quinoline and a compound of formula (I) as defined below, in the presence of a palladium based catalyst.

A second aspect of the invention is said compounds of formula I, which are novel intermediates in the process of the invention.

A third aspect of the invention is directed to a compound of formula (VI) as defined below, which are novel intermediates in the process of the invention.

A fourth aspect of the invention is directed to a compound of formula (V) as defined below, which are novel intermediates in the process of the invention.

A fifth aspect of the invention is directed to a compound of formula (VII) as defined below, which are novel intermediates in the process of the invention.

A sixth aspect of the invention is directed to a compound of formula (III) as defined below, which are novel intermediates in the process of the invention.

### DESCRIPTION OF THE INVENTION

### Process for the synthesis of Montelukast

According to a first aspect, the present invention is directed to a process for the synthesis of (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid or its salts, comprising the reaction between 7-Chloro-2-vinyl-quinoline and a compound of formula (I) 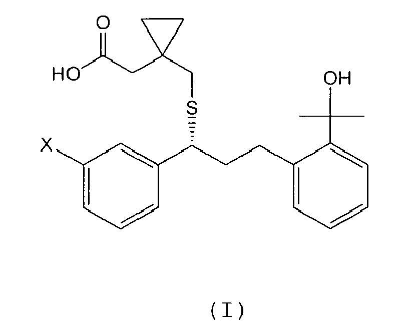 wherein X is a halogen atom or a group of formula - OSO₂R, wherein R is selected from the group consisting of CF₃, tolyl, methyl and F;
in the presence of a palladium based catalyst.

The inventors have discovered that the coupling reaction between the compounds of formula (I) and 7-Chloro-2-vinyl-quinoline proceeds without the need to protect the acid functionality of the compound of formula (I) and directly yields Montelukast. This finding provides Montelukast in good yield and saves protection/deprotection steps.

It will be immediately apparent to the skilled person that a wide range of palladium based catalysts are suitable for this reaction. The arylation of alkenes (in this case 7-Chloro-2-vinyl-quinoline) with arylhalides or arylsulfonates (in this case, the compounds of formula (I)) is commonly known by those skilled in the art as Heck and related reactions. These reactions have been extensively studied in the art. For example, see pages 930 and 931 of "Advanced Organic Chemistry. Reactions, Mechanisms, and Structure" March, J.; Smith, M. B., fifth edition, 2001, Wiley Interscience. Also in Farina, V. Adv. Synth. Catal., 2004, 346, 1553-82, a discussion of different catalyst, ligands and conditions is provided.

The reaction may proceed in homogenous and heterogeneous phase.

When the reaction is performed under homogeneous conditions, preferred catalysts are selected from the group consisting of Pd(AcO)₂, Pd(PPh₃)₄, Pd₂(dba)₃, wherein Ac is acetate, Ph is phenyl and dba is dibenzylideneacetone.

According to a preferred embodiment, the process of the invention is carried out in the presence of phosphines and diphosphines. Many phosphines and diphosphines may be found in the art (for example, see Farina, V. Adv. Synth. Catal., 2004, 346, 1553-82). The election of the most suitable phosphine or diphosphine is a matter of routine experimentation for the skilled person and fine-tunes parameters of the reaction such as the yield, the speed or the turn over of the catalyst. According to a preferred embodiment, the reaction is carried out in the presence of a phosphine or a diphosphine selected from the group consisting of tri-ortho-tolylphosphine, dppf bis(diphenylphosphino)ferrocene, BINAP (2,2'-bis(diphenylphosphino)-1,1'-binaphthyl).

Usual heterogeneous catalysts are Pd over active carbon and Pd encapsulated in organic resins or inorganic ceramics.

The reaction may proceed in the presence of a wide variety of organic solvents. For example, polar solvents such as DMF (N,N-dimethylformamide), acetonitrile, DMA (dimethylacetamide), NMP (N-methylpyrrolidone) or TMU (tetramethylurea); ethers such as DME (Dimethoxyethane), THF (Tetrahydrofuran) or dioxane; aromatic solvents such as toluene or xylenes; or alcohols. Election of the most suitable solvent requires only routine experimentation for the skilled person.

Optionally, the process of the invention may be carried out in the presence of organic bases such as tertiary amines (e.g. triethyl amine) or acetates (e.g. sodium acetate); or in the presence of inorganic bases such as carbonates (e.g. CaCO₃, Na₂CO₃ or CsCO₃) or phosphates of alcaline or alcaline earth metals (e.g. potassium phosphate).

The skilled person is aware that the reaction usually requires heat. Preferably, the reaction temperature is comprised between 30°C and 180 °C, more preferably between 50 and 120°C and more preferably between 70 and 110°C. The time required for the completion of the reaction depends on numerous factors (e.g. amount of palladium based catalyst, temperature or reactivity of the starting materials) and it is usually followed by methods known to the skilled person (e.g. chromatography or HPLC). Normal reaction times are between 1 and 48 hours, preferably under inert atmosphere.

Montelukast includes a carboxylic acid functionality and is therefore capable of forming organic and inorganic salts. Salts may have pharmacokinetic advantages with respect to the neutral form. Thus, according to a preferred embodiment, the process of the invention further comprises transforming (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid (montelukast) into a salt thereof.

According to a further preferred embodiment, said salt is sodium (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetate.

7-Chloro-2-vinyl-quinoline may be prepared by methods known in the art. For example, see the experimental section on pages 3404 and 3405 regarding the synthesis of compounds 16 and 17 in Larsen, R. D., et al J. Org. Chem. 1996, 61, 3398-3405, which is hereby incorporated by reference.

### Intermediates of formula (I), (III), (V), (VI) and (VII)

A second aspect of the invention is directed to compounds of formula (I) 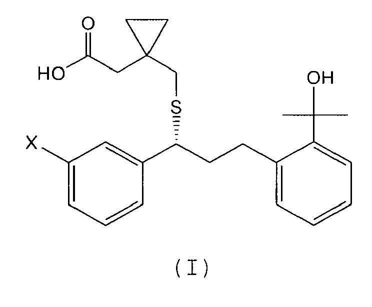 wherein X is a halogen atom or a group of formula - OSO₂R, wherein R is selected from the group consisting of CF₃, tolyl, methyl and F.

As mentioned above, said compounds of formula (I) are novel intermediates in the process of the invention.

The compounds of formula (I) may be obtained by the reaction sequence shown below in scheme 3:

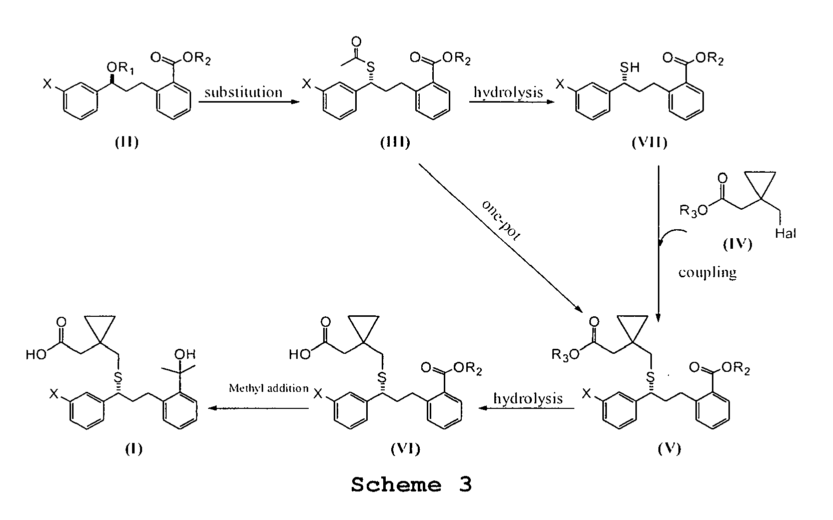

As shown in scheme 3, a preferred embodiment of invention comprises the synthesis of a the dimethylcarbinol of formula (I) by organometallic methyl addition to the aromatic carboxyester of a compound of formula (VI) 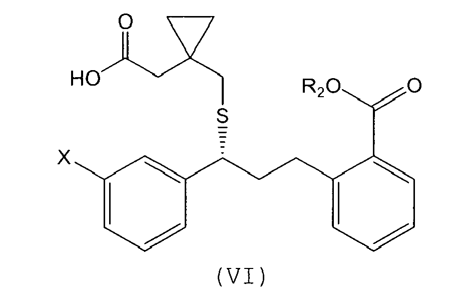 wherein X is as defined in formula (I); and
R₂ is selected from the group consisting of a C₁-C₆ alkyl.

Organometallic methyl addition conditions are known to the skilled person (for example, see pages 1214 and 1215 of "Advanced Organic Chemistry. Reactions, Mechanisms, and Structure" March, J.; Smith, M. B., fifth edition, 2001, Wiley Interscience). Preferred reagents are methyl anions of lithium or Grignard reagents, for example MeLi.LiBr, MeMgBr.CeCl₃ or MeLi. The reaction is carried out in anhydrous aprotic solvents such as THF, dioxane, DME or or hydrocarbons such as toluene or hexane or mixtures thereof.

As mentioned above, a third aspect of the invention is directed to said compounds of formula (VI), which are novel intermediates in the process of the invention.

As shown in scheme 3, a preferred embodiment of the invention comprises the synthesis of a compound of formula (VI) by hydrolysing the aliphatic ester of a compound of formula (V) 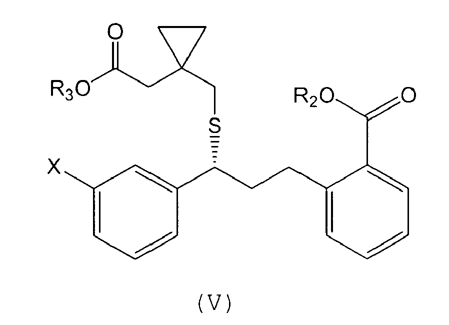 wherein
X is as defined in formula (I); and
R₂ and R₃ are independently selected from the group consisting of a C₁-C₆ alkyl.

According to a preferred embodiment, said hydrolysis is a basic hydrolysis (saponification) carried out in an organic solvent or a mixture of an organic solvent with water. Hydroxydes of alcaline metals are preferred (e.g. sodium hydroxyde).

As mentioned above, a fourth aspect of the invention is directed to said compounds of formula (V), which are novel intermediates in the process of the invention.

According to a preferred embodiment, R₃ is a C₁-C₃ alkyl group.

As shown in scheme 3, a preferred embodiment of invention comprises the synthesis of a compound of formula (V) by reacting a compound of formula (VII) 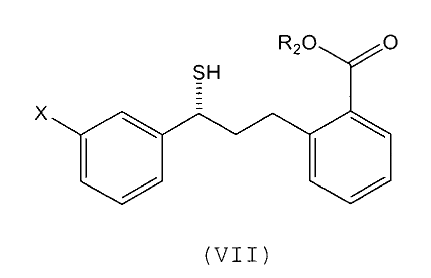 wherein
X is as defined in formula (I); and
R₂ is selected from the group consisting of a C₁-C₆ alkyl;
with a compound of formula (IV) 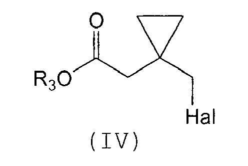 wherein
Hal is a halogen atom; and
R₃ is selected from the group consisting of a C₁-C₆ alkyl;
in the presence of a base.

According to a preferred embodiment, the base can be an organic or inorganic base. Preferably, the compound of formula (VII) and the compound of formula (IV) react in the presence of an inorganic base, such as lithium hydroxyde. Preferably, the reaction takes place in the presence of solvents, preferably, polar solvents such as alcohols (e.g. methanol, ethanol), acetonitrile or mixtures thereof.

As shown in scheme 3, a preferred embodiment of invention comprises the synthesis of a compound of formula (VII) by hydrolysing the thioester of a compound of formula (III) 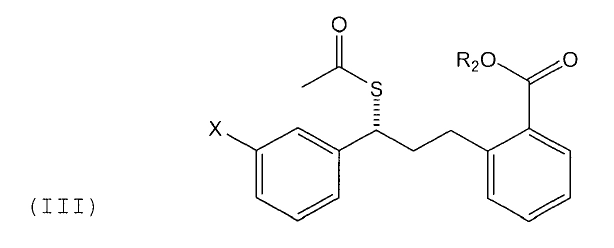 wherein
X is as defined in formula (I); and
R₂ is selected from the group consisting of a C₁-C₆ alkyl.

According to a preferred embodiment, the transformation of (III) into (V) is performed in a one-pot reaction. This embodiment eliminates the step of isolating the compound of formula (VII).

According to this embodiment, the compound of formula (IV) and the compound of formula (III) react in the presence of an inorganic base, such as lithium hydroxyde. Prefererably, the reaction takes place in the presence of polar solvents such as alcohols (e.g. methanol, ethanol), acetonitrile or mixtures thereof. Preferably the inorganic base is added in solution over the solution comprising the compound of formula (III) and the compound of formula (IV). The temperature of the reaction is preferable comprised between 0°C and reflux.

As shown in scheme 3, a preferred embodiment of invention comprises the synthesis of said compound of formula (III) by reacting a compound of formula (II) 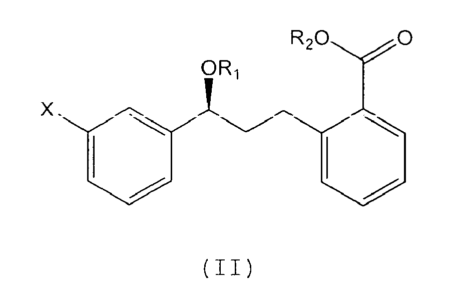 wherein
X is as defined in formula (I);
R₁ is -SO₂R, wherein R is selected from the group consisting of CF₃, tolyl, methyl and F; and
R₂ is a C₁-C₆ alkyl,
with ⁻SC (=O) CH₃.

Preferred thioacetate sources (⁻SC(=O)CH₃) are potasium thioacetate or tetramethylamonium thioacetate. The reaction may be carried out in a reaction media such as toluene, DMF, THF, isopropyl acetate, acetonitrile or mixtures thereof, at temperatures comprised between -40°C and 0°C.

The preparation of compounds of formula (II) are known in the art. 2-[3-(3-substituted-phenyl)-3-hydroxypropyl]benzoates (free hydroxyester) may be prepared from 2-[3-(3-substituted-phenyl)-3-oxopropyl]benzoates through enantioselective reduction by a method analogous to that described for the compounds of formula 3b in Larsen, R. D., et al J. Org. Chem. 1996, 61, 3398-3405 (see pages 3400 and 3403). The free hydroxyester can be further converted to a good leaving group by sulfonylation by methods known in the art to yield the compound of formula (II) (Scheme 4). For example, see page 576 of "Advanced Organic Chemistry. Reactions, Mechanisms, and Structure" March, J.; Smith, M. B., fifth edition, 2001, Wiley Interscience.

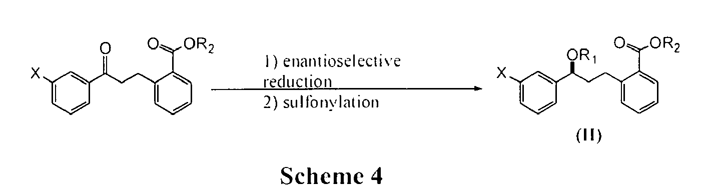

The enantioselective reduction may be further carried out by using other asymmetric catalysts. For example, (-)-DIP-Cl ((-)-B-chloroodiisopinocamphenyl borane), or with other boranes or complexes thereof with an amine using as a catalyst (R)-(+)-2-methyl-CBS-oxazaborolidine. Additionally, the free hydroxyester may be obtained by hydrogenation or hydrogen transfer of a 2-[3-(3-substituted-phenyl)-3-oxopropyl]benzoate mentioned above. A suitable catalyst may be a Ruthenium based catalyst, such as "Noyori's" catalyst (A. Fuji et al. J. Am. Chem. Soc. 1996, 118, 2521) or other catalyst wherein the metal forms a complex with chiral ligands such as chiral diphosphines (B.H. Lipshutz et al. Org. Lett. 2006, 8 (14), 2969-2972).

Polar aprotic solvents are suitable for this reaction. For example, THF (tetrahydrofuran), DMF, acetonitrile, dioxane, NMP, TMU, DME or DMA. Polar protic solvents (e.g. alcohols such as methanol, ethanol, 1-propanol, 2-propanol, etc.) may also be used. The initial reaction temperature is usually comprised between -50°C y 0°C, which is then raised to a temperature comprised between 0°C and 50°C.

Sulfonylation comprises the reaction between the free hydroxyester and a sulfonyl compound of formula RSO₂Cl, wherein R is selected from the group consisting of CF₃, tolyl, methyl and F, in the presence of an organic base, such as diisopropylethylamine(DIPEA) or triethylamine. The reaction is carried out in an aprotic solvent such as dichloromethane, preferably at a temperature comprised between -50°C and 0°C.

According to a preferred embodiment, the free hydroxyester is sulfonylated and subsequently treated with the thioacetate source in a one-pot sequence to obtain the compound of formula (III). For more details, see example 4.

According to a preferred embodiment, X is Bromine in the compounds of formula (I), (II), (III), (IV), (V), (VI) and (VII).

According to a preferred embodiment, R₂ is a C₁-C₃ alkyl group in the compounds of formula (II), (III), (V), (VI) and (VII).

### Definitions

In the above definition and in the description the following terms have the meaning indicated:
"Alkyl" refers to a hydrocarbon chain radical consisting of carbon and hydrogen atoms, containing no unsaturation, which is attached to the rest of the molecule by a single bond.
"C₁-C₆" indicates that the radical which follows comprises 1 to 6 carbon atoms (i.e., 1, 2, 3, 4, 5 or 6 carbon atoms). Analogously, "C₁-C₃" indicates that the radical comprises 1 to 3 carbon atoms (i.e., 1, 2 or 3 carbon atoms).
"halogen atom" preferably means -F, -Cl, -Br or -I.
"One-pot" refers to a reaction sequence which produces one or more intermediate compounds which, if required, could be isolated and purified, but proceeds to the final product without isolating said intermediates. For example, a reaction sequence which transforms A into B by addition of reagent X and B into the final product C by addition of reagent Y, will be considered one-pot if reagents X and Y are successively or simultaneously mixed with A in order to obtain C, without isolating B.

The following examples are displayed to illustrate the process of the present invention. They do not intend to limit in any way the scope of the invention defined in the present description.

### EXAMPLES

### Example 1

### Methyl 2-[3-(S)-(3-bromophenyl)-3-hydroxypropyl]-benzoate.

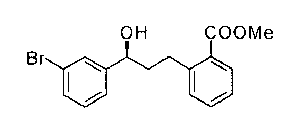

A solution of methyl 2-[3-(3-bromophenyl)-3-oxopropyl]-benzoate (4.2 g, 12.1 mmol) in THF (20 mL) is added dropwise at -30 °C to a solution of (-)-DIP-Cl (6.2 g, 18.7 mmol) in THF (30 mL), it is allowed to rise to 2.0 °C for 30 minutes and it is stirred for 3 hours until the end of the reaction (CCF; hep/EtOAc 1:1). Acetone (10 mL) is added, it is stirred for 15 minutes and poured into a mixture of saturated NH₄Cl (200 mL) and EtOAc (100 mL). The aqueous phase is extracted with more EtOAc (100 mL). The combined organic phases are washed with saturated NaCl solution (100 mL), dried (MgSO₄) and evaporated. The residue is purified by column chromatography (SiO₂) using increasing amounts of EtOAc in heptane as eluent. Evaporation of the correct fractions yields the compound of the title (3.8 g, 90%) in the form of a white solid.
¹H NMR (200 MHz, CDCl₃) : δ7.95 (d, 1H), 7.60-7.10 (m, 7H), 4.75-4.60 (m, 1H), 3.90 (s, 3H), 3.25-3.15 (m, 1H), 3.15-2-95 (m, 2H), 2.15-1.95 (m, 2H).
[α] = -37.6° (c = 1.1, CHCl₃)
Chiral HPLC S/R 98 : 2

### Example 2

### Methyl 2-[3-(R)-Acetylsulfanyl-3-(3-bromophenyl)-propyl]-benzoate.

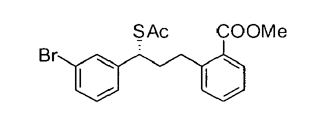

A solution of methanesulfonyl chloride (2.2 mL, 28.8 mmol) in CH₂Cl₂ (8 mL) is added dropwise at -40 °C to a solution of the hydroxy ester of the previous example (6.7 g, 19.2 mmol) and DIPEA (5.6 mL, 32.6 mmol) in CH₂Cl₂ (84 mL). After stirring for 1 hour at -10 °C, the mixture is poured on a saturated NaHCO₃ solution (150 mL). The aqueous phase is extracted with more CH₂Cl₂ (100 mL). The combined organic extracts are washed with saturated NaHCO₃ solution (75 mL), ice-cold water ( 75 mL) and saturated NaCl solution (75 mL), dried (MgSO₄) and evaporated. The raw mesylate is dissolved in toluene (83 mL) and DMF (25 mL), cooled to -10 °C and KSAc (2.28 g, 20.0 mmol) is added to it. After stirring for 3 hours at 25 °C, the mixture is diluted with toluene/EtOAc 1:1 ( 100 mL) and water (150 mL). The aqueous phase is extracted with toluene/EtOAc 1:1 ( 100 mL). The combined organic phases are washed with water (100 mL), saturated NaCl (100 mL), dried (MgSO₄) and evaporated. The residue is purified by column chromatography (SiO₂) using increasing amounts of EtOAc in heptane as eluent. Evaporation of the correct fractions yields the compound of the title (6.1 g, 78%) in the form of a yellowish oil.
¹H NMR (200 MHz, CDCl₃) : δ 7.85 (d, 1H), 7.55-7.10 (m, 7H), 4.58 (t, 1H), 3.90 (s, 3H), 3.15-2.75 (m, 2H), 2.40-2.10 (m, 5H) includes 2.30(s, 3H).
¹³C NMR (50 MHz, CDCl₃) : δ 194.1, 167.6, 144.0, 142.5, 131.9, 130.9, 130.7, 130.5, 130.2, 129.9, 129.2, 126.3, 126.1, 122.4, 51.8, 47.3, 37.3, 32.5, 30.3.
[α] = +137.2° (c = 1.4, CHCl₃)
Chiral HPLC S/R 0.2 : 99.8

### Example 3

### Methyl 2-[3-(R)-(3-bromophenyl)-3-mercaptopropyl]-benzoate

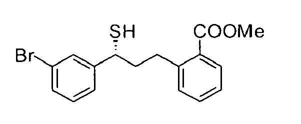

A solution of the thioacetate of example 2 (5.9 g, 14.5 mmol) in MeOH/CH₃CN 2:1 (120 mL) is degassed by passing a nitrogen stream for 1 hour. A solution of LiOH.H₂O (0.61 g, 14.5 mmol) in water (5 mL) is added dropwise and the mixture is stirred at 20 °C for 5 hours. It is poured on EtOAc (250 mL) and water (100 mL) containing AcOH (15.0 mmol), the aqueous phase is extracted with EtOAc (100 mL), the combined organic phases are washed with water (100 mL) and saturated NaCl solution (100 mL). After drying with MgSO₄, it is evaporated, and the residue is purified by column chromatography (SiO₂) using increasing amounts of EtOAc in heptane as eluent. The evaporation of the correct fractions yields the compound of the title (4.7 g, 89%) in the form of a colorless oil.
¹H NMR (200 MHz, CDCl₃): δ 7.85 (d, 1H), 7.55-7.10 (m, 7H), 4.05-3.90 (m, 1H), 3.86 (s, 3H), 3.20-3.00 (m, 1H), 3.00-2.80 (m, 1H), 2.30-2.10 (m, 1H), 2.00 (d, 1H).
¹³C NMR (50 MHz, CDCl₃): δ 167.9, 147.1, 143.0, 132.2, 131.2, 131.0, 130.4, 130.3, 130.2, 129.4, 126.4, 125.8, 122.7, 52.1, 43.6, 41.3, 33.1.

### Example 4

### Methyl 2-[3-(R)-(3-Bromophenyl)-3-(1-methoxycarbonylmethyl-cyclopropylmethylsulfanyl)-propyl]-benzoate.

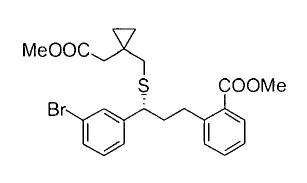

A solution of the thioacetate of example 2 (5.9 g, 14.5 mmol) and methyl (1-bromomethyl-cyclopropyl)-acetate (4.5 g, 21.7 mmol) in MeOH/CH₃CN 2:1 (120 mL) is degassed by passing a nitrogen stream for 1 hour. A solution of LiOH.H₂O (0.61 g, 14.5 mmol) in water (5 mL) is added dropwise and the mixture is stirred at 20 °C for 5 hours. It is poured on EtOAc (250 mL) and water (100 mL), the aqueous phase is extracted with EtOAc (100 mL), the combined organic phases are washed with water (100 mL) and saturated NaCl solution (100 mL). After drying with MgSO₄, it is evaporated, and the residue is purified by column chromatography (SiO₂) using increasing amounts of EtOAc in heptane as eluent. The evaporation of the correct fractions yields the compound of the title (4.9 g, 69%) in the form of a yellowish oil.
¹H NMR (200 MHz, CDCl₃) : δ 7.85 (d, 1H), 7.55-7.10 (m, 7H), 3.90 (s, 3H), 3,80 (t, 1H), 3.60(s, 3H), 3.15-2.75 (m, 2H), 2.45 (s, 2H), 2.38(d, 2H), 2.25-1.95 (m, 2H), 0.60-0.30 (m, 4H).
¹³C NMR (50 MHz, CDCl₃): δ 172.5, 167.8, 145.4, 143.0, 132.0, 131.0, 130.8, 130.1, , 129.9, 129.4, 126.7, 126.1, 122.5, 51.9, 51.4, 49.6, 39.9, 39.2, 38.5, 32.7, 16.8, 12.7, 12.3.
[α] = +91.7° (c = 1.05, CHCl₃)
Chiral HPLC S/R 0.5 : 99.1

### Example 5

### Methyl 2-[3-(R)-(3-Bromophenyl)-3-(1-carboxymethyl-cyclopropylmethylsulfanyl)-propyl]-benzoate.

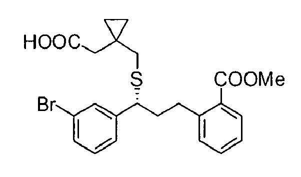

A solution of 1N NaOH (14.4 mL, 14.4 mmol) is added dropwise to a solution of the product of the previous example (4.7 g, 9.6 mmol) in MeOH/ THF 1:1 (100 mL) and the mixture is stirred for 15 hours at 20 °C. The solvent is evaporated and the residue is distributed between water (100 mL) and EtOAc (100 mL). The aqueous phase is extracted with EtOAc (50 mL).
The combined organic extracts are washed with saturated NaCl solution (50 mL), dried (MgSO₄) and evaporated. The residue is purified by column chromatography (SiO₂) using increasing amounts of EtOAc in heptane containing 0.1% of AcOH as eluent. The evaporation of the correct fractions yields the compound of the title (2.8 g, 61%) in the form of a colorless oil.
¹H NMR (200 MHz, CDCl₃) : δ 7.90 (d, 1H), 7.55-7. 10 (m, 7H), 3.90-3.70 (m, 4H) includes 3.90 (s, 3H), 3.20-2.75 (m, 2H), 2.50-2.35(m, 4H), 2.25-1.95 (m, 2H), 0.60-0.30 (m, 4H).
¹³C NMR (50 MHz, CDCl₃): δ 178.1, 167.9, 145.4, 143.0, 132.0, 131.0, 130.8, 130.2, 130.0, 129.4, 126.6, 126.1, 122.5, 51.9, 49.6, 39.8, 39.0, 38.5, 32.7, 16.6, 12.7, 12.3.
[α] = +101.2° (c = 0.73, CHCl₃)
Chiral HPLC S/R 0.2 : 99.7

### Example 6

### (1-{1-(R)-(3-Bromophenyl)-3-[2-(1-hydroxy-1-methyl-ethyl)-phenyl]-propylsulfanylmethyl}-cyclopropyl)-acetic acid.

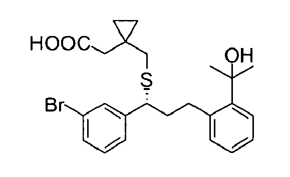

A 1.5M solution of the MeLi.LiBr complex in diethyl ether (15 mL, 22.5 mmol) is added dropwise at -40 °C to a solution of the compound of the previous example (2.6 g, 5.4 mmol) in dry THF (30 mL). The bath is withdrawn, it is stirred at 20 °C for 15 hours and it is poured on a mixture of saturated NH₄Cl solution (200 mL) and EtOAc (100 mL). The aqueous phase is extracted with EtOAc (100 mL). The combined organic extracts are washed with saturated NaCl solution (50 mL), dried (MgSO₄) and evaporated. The residue is purified by column chromatography (SiO₂) using increasing amounts of EtOAc in heptane containing 0.1% of AcOH as eluent. The evaporation of the correct fractions yields the compound of the title (1.8 g, 69%) in the form of a yellowish oil.
¹H NMR (200 MHz, CDCl₃): δ 7.55 (bs, 1H), 7.40-7.00 (m, 7H), 4.60 (bs, 2H, exchanges with D20), 3.90 (t, 1H), 3.25-3.05 (m, 1H), 2.90-2.70 (m, 1H), 2.60-2.25(m, 4H), 2.25-1.95 (m, 2H), 1.60 (2s, 6H), 0.60-0.30 (m, 4H).
¹³C NMR (50 MHz, CDCl₃) : δ 177.7, 145.6, 145.1, 139.9, 131.4, 130.8, 130.1, 130.0, 127.1, 126.6, 125.7, 125.4, 125.2, 122.5, 73.9, 49.6, 40.0, 39.8, 39.1, 32.1, 31.7, 16.6, 12.8, 12.2.
[α] = +117.1° (c = 0.55, CHCl₃)
Chiral HPLC S/R 0.3 : 99.6

### Example 7

### (1-{1-(R)-{3-[2-(7-Chloroquinoline-2-yl)-vinyl]-phenyl}-3-[2-(1-hydroxy-1-methyl-ethyl)-phenyl]-propylsulfanylmethyl}-cyclopropyl)-acetic acid.

A mixture of the compound of the previous example (800 mg, 1.7 mmol), 7-chloro-2-vinylquinoline ( 381 mg, 2.0 mmol), tri(o-tolyl)phosphine (243 mg, 0.8 mmol) and Pd(OAc)₂ (45 mg, 0.2 mmol) in dry DMF (4 mL) is degassed by bubbling nitrogen for 15 minutes. NEt₃ ( 0.7 mL, 5.0 mmol) is added and it is heated at 100 °C for 4 hours. After cooling, its is poured on a mixture of tol/EtOAc 1:1 (50 mL) and water (50 mL). The pH is adjusted to 3-4 with citric acid and the aqueous phase is extracted with more (50 mL) tol/EtOAc 1:1. The combined organic extracts are washed with saturated NaCl solution (50 mL), dried (MgSO₄) and evaporated. The residue is purified by column chromatography (SiO₂) using increasing amounts of EtOAc in heptane containing 0.1% of AcOH as eluent. The evaporation of the correct fractions yields the compound of the title (850 mg, 85%) in the form of a yellow solid.
¹H NMR (200 MHz, CDCl₃): δ 8.20-8.05 (m, 2H), 7.85-7.00 (m, 13H), 4.60 (bs, 2H, exchanges with D20), 4.02 (t, 1H), 3.30-3.05 (m, 1H), 3.05-2.80 (m, 1H), 2.70-2.10(m, 6H), 1.63 (s, 6H), 0.70-0.35 (m, 4H).
¹³C NMR (50 MHz, CDCl₃): δ 176.4, 156.9, 148.0, 145.2, 143.6, 140.1, 136.4, 136.3, 135.7, 135.5, 131.5, 129.0, 128.7, 128.6, 128.4, 128.3, 128.2, 127.5. 127.2, 127.1, 126.7, 126.4, 125.6, 125.4, 125.3, 119.1, 73.8, 50.3, 40.2, 39.9, 38.9, 32.3, 31.8, 16.8, 12.7, 12.4.
[α] = +62.1° (c = 1.1, CHCl₃)
Chiral HPLC S/R 0.3 : 99.7

## Claims

1. Process for the synthesis of (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid or its salts, comprising the reaction between 7-Chloro-2-vinyl-quinoline and a compound of formula (I) 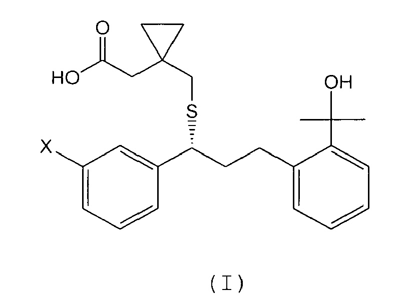 wherein X is a halogen atom or a group of formula - OSO₂R, wherein R is selected from the group consisting of CF₃, tolyl, methyl and F;
in the presence of a palladium based catalyst.

2. Process according to claim 1, wherein the reaction is carried out in the presence of a phosphine or a diphosphine.

3. Process according to any of the previous claims, wherein the reaction is carried out in the presence of an organic or an inorganic base.

4. Process according to any of the previous claims, wherein the palladium catalyst is an homogeneous or heterogeneous catalyst selected from the group consisting of Pd(AcO)₂, Pd(PPh₃)₄, Pd₂(dba)₃, Pd over active carbon and Pd encapsulated in organic resins or inorganic ceramics.

5. Process according to any of the previous claims, wherein the reaction takes place in the presence of a polar solvents selected from the group consisting of N,N-dimethylformamide, acetonitrile, dimethylacetamide, N-methylpyrrolidone and tetramethylurea; dimethoxyethane ; an aromatic solvent, an alcohol or mixtures thereof.

6. Process according to any of the previous claims, wherein the reaction is carried out at a temperature comprised between 30 and 180°C.

7. Process according to any of the previous claims, wherein said compound of formula (I) is obtained by organometallic methyl addition to the aromatic carboxyester of a compound of formula (VI) 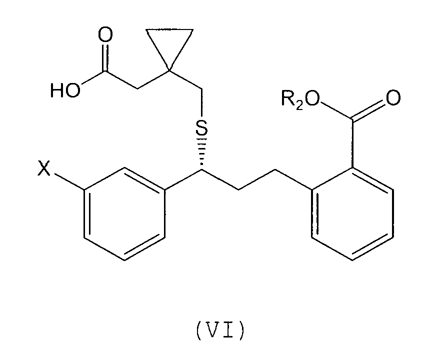 wherein X is as defined in claim 1; and
R₂ is selected from the group consisting of a C₁-C₆ alkyl.

8. Process according to claim 7 wherein said compound of formula (VI) is obtained by hydrolysing the aliphatic ester of a compound of formula (V) 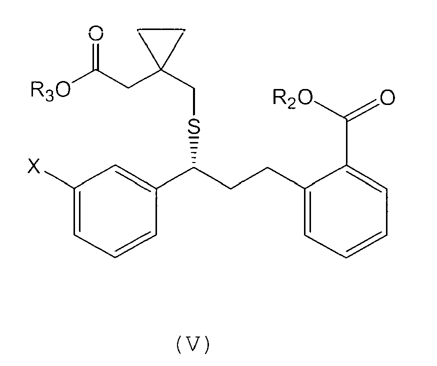 wherein
X is as defined in claim 1; and
R₂ and R₃ are independently selected from the group consisting of a C₁-C₆ alkyl.

9. Process according to claim 8, wherein said compound of formula V is obtained by reacting a compound of formula (VII) 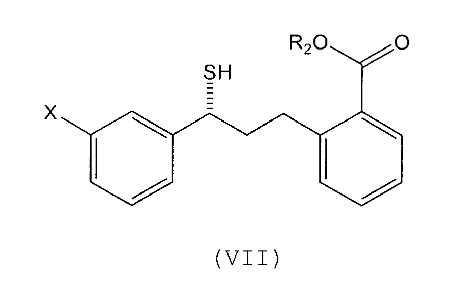 wherein
X is as defined in claim 1; and
R₂ is selected from the group consisting of a C₁-C₆ alkyl;
with a compound of formula (IV) 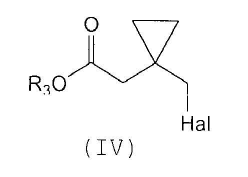 wherein
Hal is a halogen atom; and
R₃ is selected from the group consisting of a C₁-C₆ alkyl;
in the presence of a base.

10. Process according to claim 9, wherein said compound of formula (VII) is obtained by hydrolysing the thioester of a compound of formula (III) 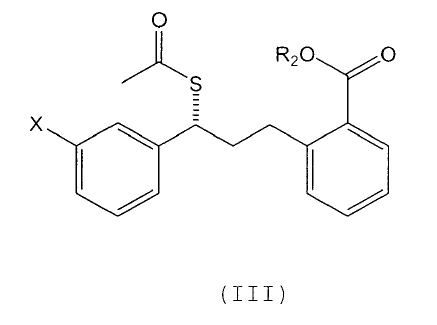 wherein
X is as defined in claim 1; and
R₂ is selected from the group consisting of a C₁-C₆ alkyl.

11. Process according to 9 and 10, wherein the transformation of (III) into (V) is performed in a one-pot reaction.

12. Process according to any of claims 10 or 11, wherein said compound of formula (III) is obtained by reacting a compound of formula (II) 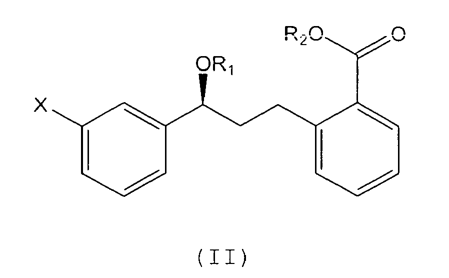 wherein
X is as defined in claim 1;
R₁ is -SO₂R, wherein R is selected from the group consisting of CF₃, tolyl, methyl and F; and
R₂ is a C₁-C₆ alkyl,
with ⁻SC(=O)CH₃.

13. Process according to any of the previous claims, wherein (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid is further transformed into a salt thereof.

14. Process according to claim 13, wherein (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid is further transformed into sodium (1-{1-(R)-(E)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetate.

15. A compound of formula (I) 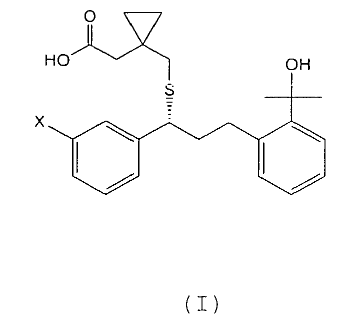 wherein
X is a halogen atom or a group of formula -OSO₂R, wherein R is selected from the group consisting of CF₃, tolyl, methyl and F.

16. A compound of formula (VII) 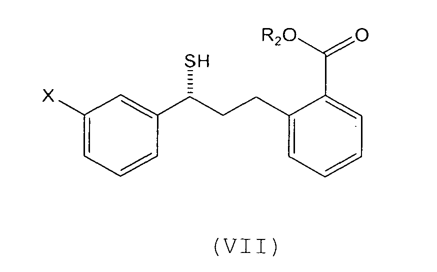 wherein
X is as defined in claim 1; and
R₂ is selected from the group consisting of a C₁-C₆ alkyl;

17. A compound of formula (III) 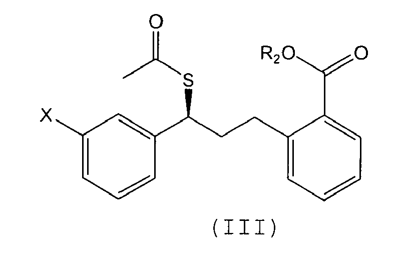 wherein
X is as defined in claim 1; and
R₂ is selected from the group consisting of a C₁-C₆ alkyl;
or its salts thereof.

18. A compound of formula (V) 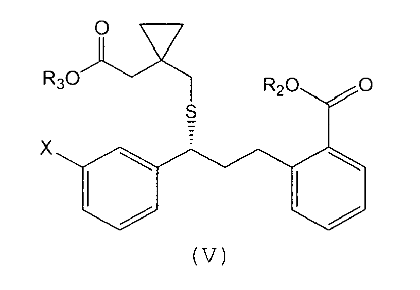 wherein
X is as defined in claim 1; and
R₂ and R₃ are independently selected from the group consisting of a C₁-C₆ alkyl;
or its salts thereof.

19. A compound according to claim 18, wherein R₃ is a C₁-C₃ alkyl group.

20. A compound of formula (VI) 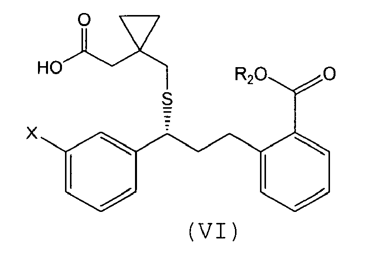 wherein
X is as defined in claim 1; and
R₂ is selected from the group consisting of a C₁-C₆ alkyl;
or its salts thereof.

21. A compound according to any of claims 15 to 20, wherein X is Bromine.

22. A compound according to any of claims 16 to 21, wherein R₂ is a C₁-C₃ alkyl group.
